# EUROPÄISCHE PATENTSCHRIFT

(11) **EP 0 552 632 B1**
(45) Veröffentlichungstag und Bekanntmachung des Hinweises auf die Patenterteilung: **13.03.1996**
(21) Anmeldenummer: 93100274.5
(22) Anmeldetag: 11.01.1993
(51) Int. Cl.: C07C 249/02, C07C 251/20

(54) **Verfahren zur Herstellung von Azomethinen**
Process for the preparation of azomethins
Procédé de préparation d'azométhines

(30) Priorität: 22.01.1992 DE 4201605
(43) Veröffentlichungstag der Anmeldung: 28.07.1993
(73) Patentinhaber: BAYER AG, D-51368 Leverkusen (DE)
(72) Erfinder: Langer, Reinhard, Dr., W-4150 Krefeld (DE); Buysch, Hans-Josef, Dr., W-4150 Krefeld (DE); Wagner, Paul, Dr., W-4000 Düsseldorf 13 (DE)

(56) Entgegenhaltungen:
- EP-A- 0 014 264
- EP-A- 0 470 460
- DE-A- 2 166 941
- FR-A- 2 313 354
- BERICHTE DER DEUTSCHEN CHEMISCHEN GESELLSCHAFT Bd. 53, 1920, WEINHEIM DE Seiten 345-354 G. REDDELIEN 'Über Kondensationsprodukte zwischen Anilin und hydro-aromatischen Ketonen.'

## Beschreibung

Die Erfindung betrifft ein Verfahren zur Herstellung von N-Cycloalkylidenanilinen durch sauer katalysierte Wasserabspaltung und azeotrope Entfernung des Reaktionswassers.

Das Verfahren beinhaltet die Umsetzung von gegebenenfalls kernsubstituierten Anilinen mit gegebenenfalls substituierten Cycloalkanonen in Gegenwart mindestens eines sauren in Anilin-Cycloalkanon-Azomethin-Mischungen löslichen Katalysators bei erhöhter Temperatur in einer Destillationskolonne.

Die Kondensation von aromatischen Aminen mit Cycloalkanonen ist eine Gleichgewichtsreaktion mit geringer Wärmetönung. Sie wird zugunsten des Azomethins (Schiff'sche Base) verschoben, wenn man dem System das entstehende Reaktionswasser entzieht. Im allgemeinen erreicht man dies durch azeotrope Destillation, wozu gegebenenfalls ein inertes Schleppmittel, wie Benzol, Toluol usw. eingesetzt wird.

In jüngster Zeit wurde zur Bindung des Reaktionswassers der Einsatz von TiCl₄ (J. Org. Chem. 32 (1967), 3247) oder von molaren Mengen (Butyl)₂SnCl₂ (Synth. Commun. 12 (1982), 495) beschrieben. Beide Verbindungen binden das entstehende Reaktionswasser unter Freisetzung von HCl.

Andere Entwicklungen gingen dahin, daß die das Wasser bindende Spezies kovalent an das Anilin gebunden ist, beispielsweise in Form von N,N-Bis-(trimethylsilyl)-anilin (Bull. Soc. Chim. Fr. 1966, 3205), Iminophosphoranen (Angew. Chem. Int. Ed. Eng. 5 (1966), 947) sowie N-(Diphenylaluminium)-anilin (J. Org. Chem. 51 (1986), 1848).

Eine weitere Methode, das entstehende Reaktionswasser wirkungsvoll zu binden und so bei geringen Anilin- oder Ketonüberschüssen unter milden Bedingungen arbeiten zu können, ist der Einsatz von Molekularsieben (J. Org. Chem. 36 (1971), 1570; DE-OS 2 244 238). Der Nachteil dieses zuletzt genannten Verfahrens ist die aufwendige und kostspielige Regenerierung des Molekularsiebs.

Die azeotrope Entwässerung ist sicherlich das technisch interessanteste Verfahren, wenn es gelingt, die Umsetzung unter geringem Energieeinsatz mit guter Raum-Zeit-Ausbeute und Selektivität und ohne größere Mengen an Azeotrop-Schleppmittel in einer einfachen Apparatur durchzuführen.

Hohe Raum-Zeit-Ausbeuten haben eine große Reaktionsgeschwindigkeit und damit eine effektive Katalyse zur Voraussetzung.

Die Kondensation von Cyclohexanon und Anilin zu Cyclohexylidenanilin mit Zinkchlorid als Katalysator ist seit langem bekannt (Ber. 53 (1920) 345-354). Für schwierige Fälle in solchen Kondensationen wurde das Katalysatorsystem HCl-ZnCl₂ eingesetzt (Ber. 46 (1913) 2718).

In DE-AS 1 078 119 wird die Kondensation von N-Phenyl-p-phenylen-diamin mit Cyclohexanon ohne Katalysatorzusatz beschrieben; hierbei müssen Cyclohexanonüberschüsse von 200 bis 300 % eingesetzt werden.

In DE-OS 2 525 295 wird gezeigt, daß die Reaktionszeit der Kondensation von Anilin mit 400 % Überschuß Cyclohexanon ohne Katalysator mit steigender Ansatzgröße stark zunimmt, so daß eine Übersetzung in den technischen Maßstab nicht möglich ist. Ferner wurde gezeigt, daß stark- und schwachsaure organische Harze einen günstigen Einfluß auf die Reaktionszeit besitzen.

In DE-OS 2 901 863 werden frisch synthetisiertes, wasserfreies, nicht gebranntes Calciumhydrogenphosphat, Apatit der Formel Ca₅(PO₄)₃OH, getrocknete, nicht geglühte Aluminiumoxidhydroxide und protonengetauschte, neutral gewaschene Aluminiumsilikate vom Montmorillonit-Typ als wirkungsvolle Katalysatoren für die Umsetzung von aromatischen Aminen mit Ketonen beschrieben. Die Beispiele in dieser Patentanmeldung beschränken sich jedoch auf die Kondensation des reaktiven p-Phenylendiamins mit Methyl-isobutylketon, wobei ein Ketonüberschuß von 150 % angewandt wird.

Alle genannten Verfahren zur Azomethinsynthese durch azeotrope Entwässerung leiden darunter, daß die Vervollständigung des Umsatzes zugleich langwierig und energetisch aufwendig ist.

Es war somit wünschenswert, ein preiswertes und umweltschonendes Verfahren zur Synthese von Cycloalkylidenanilinen zu entwickeln, welches sich durch hohe Ausbeuten, eine einfache Apparatur und optimale Energieausnutzung bei minimalem Energieeinsatz auszeichnet.

Gefunden wurde ein Verfahren, bei dem die Edukte mit einem minimalen Überschuß an Carbonyl- oder Anilinkomponente in einen kontinuierlich arbeitenden Reaktor eingespeist werden, aus dem die Azomethinverbindung mit hoher Ausbeute und in hoher Reinheit direkt entnommen werden kann.

Es wurde ein Verfahren zur Herstellung von Azomethinen der Formel 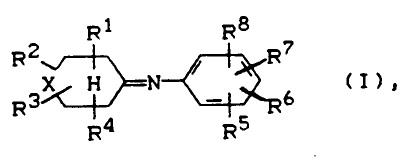
in der
- R¹, R², R³, R⁴, R⁵, R⁶, R⁷ und R⁸: unabhängig voneinander Wasserstoff, geradkettiges oder verzweigtes C₁-C₆-Alkyl, C₃-C₆-Cycloalkyl oder Aryl bedeuten und
- R⁵, R⁶, R⁷ und R⁸: unabhängig voneinander zusätzlich Halogen, geradkettiges oder verzweigtes C₁-C₆-alkoxy, Hydroxy, Amino, C₁-C₆-Alkylamino, Di-C₁-C₆-alkyl-amino, Aryloxy oder Arylamino bedeuten,
wobei Aryl für Phenyl oder für in 2-, 3- oder 4-Stellung gebundenes 5- oder 6-gliedriges Heteroaryl mit 1 oder 2 Heteroatomen aus der Gruppe N, O und S steht, und
X -CH₂- oder eine zwischen den benachbarten C-Atomen durchgehende Bindung bedeutet,
durch Kondensation von Cycloalkanonen der Formel 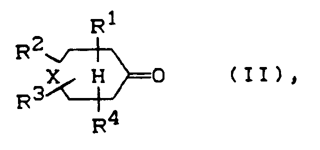
mit Anilinen der Formel 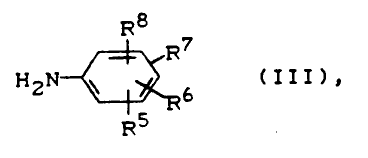
in denen R¹ bis R⁸ und X die obige Bedeutung haben, gefunden, das dadurch gekennzeichnet ist, daß die Kondensation in kontinuierlicher Reaktion in einer Destillationskolonne in Gegenwart von sauren homogenen Katalysatoren mit einem in H₂O gemessenen pKₐ-Wert von 1-6 unter azeotroper Entfernung des Reaktionswassers durchgeführt wird, wobei die Ausgangsstoffe, der Azeotropbildner und der Katalysator oder die Katalysatormischung getrennt an verschiedenen Orten oder zusammen als Mischung am gleichen Ort in die Destillationskolonne eingespeist werden und von den Reaktionsprodukten das azeotrop zu entfernende Wasser aus dem Niedertemperaturbereich und das entstehende Azomethin aus dem Hochtemperaturbereich abgeführt werden.

In bevorzugter Weise werden Ausgangsstoffe eingesetzt, in denen Phenyl an Stelle von Aryl steht.

In weiterhin bevorzugter Weise werden Cycloalkanone eingesetzt, in denen R⁴ Wasserstoff bedeutet.

In noch weiterhin bevorzugter Weise werden Aniline eingesetzt, in denen R⁸ Wasserstoff bedeutet.

In besonders bevorzugter Form werden Cycloalkanone eingesetzt, in denen R³ und R⁴ Wasserstoff bedeuten.

In ebenfalls besonders bevorzugter Weise werden Aniline eingesetzt, in denen R⁷ und R⁸ Wasserstoff bedeuten.

Des weiteren sind Cycloalkanone der Formel 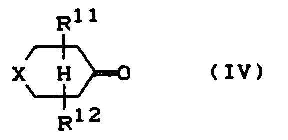
bevorzugt, in der
- R¹¹ und R¹²: unabhängig voneinander Wasserstoff, geradkettiges oder verzweigtes C₁-C₄-Alkyl, Cyclopropyl, Cyclopentyl, Cyclohexyl oder Phenyl bedeuten und
X -CH₂- oder eine zwischen den benachbarten C-Atomen durchgehende Bindung bedeutet.

In besonders bevorzugter Weise werden Cycloalkanone der Formel 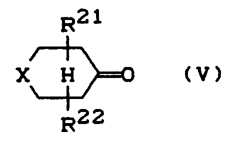
eingesetzt, worin
- R²¹ und R²²: unabhängig voneinander Wasserstoff, Methyl oder Ethyl bedeuten und
- X: die obige Bedeutung hat.

In bevorzugter Weise werden weiterhin Aniline der Formel 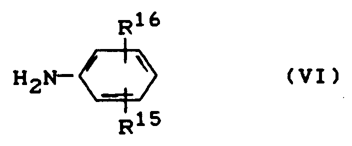
eingesetzt, in der
- R¹⁵ und R¹⁶: unabhängig voneinander Wasserstoff, geradkettiges oder verzweigtes C₁-C₄-Alkyl, Phenyl, Fluor, Chlor, Brom, geradkettiges oder verzweigtes C₁-C₄-Alkoxy, Hydroxy, Amino, C₁-C₄-Alkylamino, Di-C₁-C₄-alkyl-amino, Phenoxy oder Phenylamino bedeuten.

In besonders bevorzugter Weise werden Aniline der Formel 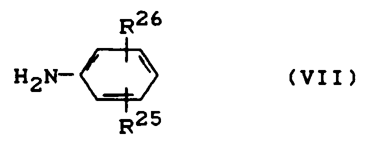
eingesetzt, in der
- R²⁵ und R²⁶: unabhängig voneinander Wasserstoff, Methyl, Ethyl, Fluor, Chlor, Methoxy, Ethoxy, Methylamino, Ethylamino, Dimethylamino oder Diethylamino bedeuten.

In ganz besonders bevorzugter Weise werden Aniline der Formel 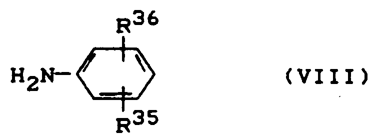
eingesetzt, in der
- R³⁵ und R³⁶: unabhängig voneinander Wasserstoff, Fluor, Chlor, Methyl, Ethyl, Methoxy, Methylamino oder Dimethylamino bedeuten.

Als Alkylgruppen in den genannten Substituenten (Alkyl, Alkoxy, Alkylamino, Dialkylamino) seien geradkettige oder verzweigte C₁-C₆-Alkylgruppen genannt, wie Methyl, Ethyl, Propyl, Isopropyl, Butyl, Isobutyl, tert.-Butyl, die isomeren Pentyle und Hexyle. In bevorzugter Weise seien die genannten C₁-C₄-Alkylgruppen genannt, besonders bevorzugt Methyl und Ethyl, ganz besonders bevorzugt Methyl.

Als Aryl in den genannten Substituenten (Aryl, Aryloxy, Arylamino) seien Phenyl oder ein in 2-, 3- oder 4-Stellung gebundenes 5- oder 6-gliedriges Heteroaryl mit 1 oder 2 Heteroatomen aus der Gruppe N, O, S genannt. Beispiele für solches Heteroaryl sind: Furanyl, Pyrrolyl, Thienyl, Pyrazolyl, Imidazolyl, Oxazolyl, Thiazolyl, Pyridyl und andere. In bevorzugter Weise steht Phenyl an Stelle von Aryl.

Als C₃-C₆-Cycloalkyl sei beispielsweise Cyclopropyl, Cyclobutyl, Cyclopentyl und Cyclohexyl, bevorzugt Cyclopropyl, Cyclopentyl und Cyclohexyl, genannt. Die Cycloalkyl-Substituenten können ihrerseits ein- oder zweimal durch Methyl oder Ethyl substituiert sein.

Als Halogen sei beispielsweise Fluor, Chlor, Brom, Iod, bevorzugt Fluor, Chlor, Brom, besonders bevorzugt Fluor und Chlor, genannt.

Der Ausdruck "X" im Cycloalkylring in den Formeln (I), (II), (IV) und (V) kann die Methylengruppe -CH₂- oder eine zwischen den benachbarten C-Atomen durchgehende Bindung bedeuten. Im ersteren Fall wird hiermit das Cyclohexangerüst, im zweiten Fall das Cyclopentangerüst ausgedrückt. In bevorzugter Weise bedeutet X die Methylengruppe und stellt somit das Cyclohexangerüst dar.

Folgende Cycloalkanone seien als Ausgangsverbindungen im einzelnen beispielhaft aufgeführt:
Cyclopentanon, 2-Methyl-cyclopentanon, 2-Ethyl-cyclopentanon, 2-Phenyl-cyclopentanon, Cyclohexanon, 2-Methyl-cyclohexanon, 4-Methyl-cyclohexanon, 2-Ethyl-cyclohexanon, 4-Ethyl-cyclohexanon, 4-Phenyl-cyclohexanon, 2-Phenyl-cyclohexanon, 4-Cyclohexyl-cyclohexanon, 2-Cyclohexyl-cyclohexanon.

Folgende Aniline als Ausgangsverbindungen seien im einzelnen beispielhaft aufgeführt:
Anilin, 2-Methylanilin, 3-Methylanilin, 4-Methylanilin, 2,4-Dimethyl-anilin, 2-Ethylanilin, 4-Ethylanilin, 3-Methoxyanilin, 3-Ethoxyanilin, 4-Aminoanilin, 4-Methylamino-anilin, 4-Dimethylamino-anilin, 4-Phenylanilin, 2-Phenylanilin, 4-Phenoxyanilin, 2-Phenoxyanilin, 4-Phenylamino-anilin, 2-Phenylamino-anilin, 2-Fluoranilin, 4-Fluoranilin, 2-Chloranilin, 4-Chloranilin.

Das Molverhältnis, in welchem das Cycloalkanon und das Anilin zudosiert werden, beträgt von 2:1 bis 1:2, bevorzugt von 1,5:1 bis 1:1,5, besonders bevorzugt von 1,2:1 bis 1:1,2, ganz besonders bevorzugt von 1,1:1 bis 1:1,1.

Zur azeotropen Entfernung des Reaktionswassers können Azeotropbildner aus der Gruppe der aliphatischen und aromatischen Kohlenwasserstoffe, der aliphatischen und aromatischen Ether sowie der aliphatischen Alkohole eingesetzt werden. In diesen Verbindungen können Wasserstoffatome durch Halogenatome ersetzt sein, Diese Azeotropbildner, ihre Wirkungsweise und ihr Einsatz sind dem Fachmann grundsätzlich bekannt Als Azeotropbildner seien beispielhaft folgende aufgeführt:
Benzol, Toluol, Xylol, Anisol, Chlorbenzol, Butanol, Hexanol, Methyl-butylether, Diphenylether, p-Chloranisol. Das Gewichtsverhältnis, in dem ein Azeotropbildner und die Summe der Ausgangsstoffe zudosiert werden, beträgt 0,001-1:1, bevorzugt 0,001-0,5:1, besonders bevorzugt 0,001-0,2:1, ganz besonders bevorzugt 0,001-0,1:1. In einer dem Fachmann bekannten Weise kann das abdestillierte Azeotrop außerhalb des kolonnenartigen Reaktors kondensiert und in Reaktionswasser und Azeotropbildner geschieden werden, Der so zurückerhaltende Azeotropbildner kann erneut in die Reaktion des erfindungsgemäßen Verfahren zurückgeführt werden.

Als Azeotropbildner kann auch einer der Ausgangsstoffe, bevorzugt das Cycloalkanon dienen, so daß auf einen systemfremden Azeotropbildner verzichtet werden kann.

Als saure homogene Katalysatoren kommen prinzipiell alle anorganischen oder organischen Verbindungen in Frage, die einen in H₂O gemessenen pKₐ-Wert von 1-6, vorzugsweise 2-5,5, besonders bevorzugt 3-5, aufweisen. Bevorzugt werden Alkyl- oder Arylcarbonsäuren in reiner Form oder als Mischungen eingesetzt.

Besonders bevorzugt werden C₁₋₁₀-, ganz besonders bevorzugt C₂₋₆-Carbonsäuren in reiner Form oder als Mischungen eingesetzt.

Beispielsweise seien genannt: Ameisensäure, Essigsäure, Propionsäure, Buttersäure, Benzoesäure, Valeriansäure und Isobuttersäure.

Betrachtet man die Summe der sauren Gruppen der dosierten katalytisch aktiven Säuren im Verhältnis zur Summe der dosierten Eduktmoleküle, so bewegt sich die dosierte Säuremenge im Bereich von 10 bis 0,001 Mol-%, bevorzugt von 1 bis 0,01 Mol-%, besonders bevorzugt von 0,5 bis 0,02 Mol-%.

Es werden dabei Säuremischungen verwendet, die zu wenigstens 50 %, bevorzugt zu wenigstens 70 %, besonders bevorzugt zu wenigstens 90 % mit der azeotropen Gasphase dem Reaktor aus dem Niedertemperaturbereich wieder entnommen werden.

Dies ist deswegen günstig, weil große Mengen an Säure im Hochtemperaturbereich zur Kondensation von Azomethinmolekülen unter Anilinabspaltung Anlaß geben können und demnach von diesem Reaktorbereich weitgehend ferngehalten werden sollten. Hierzu wird im erfindungsgemäßen Verfahren der im Reaktor ablaufende Destillationsvorgang benutzt; entsprechend ist für jede der beanspruchten Substratkombinationen aus den beanspruchten Säurekombinationen im Einzelfall eine Optimale experimentell zu ermitteln.

Das erfindungsgemäße Verfahren ist dadurch gekennzeichnet, daß die Kondensation in einer für den kontinuierlichen Betrieb geeigneten Destillationsapparatur durchgeführt wird.

Eine derartige Apparatur besteht im einfachsten Fall aus einer isolierten Kolonne mit Kondensator und Strömungsteiler am Kolonnenkopf (Niedertemperaturbereichsende) und einem Verdampfer beliebiger Ausführung mit kontinuierlicher Produktausschleusung am Kolonnenfuß (Hochtemperaturbereichsende der Destillationsapparatur).

Die Kolonne kann, entsprechend den in ihr ablaufenden Vorgängen, definitionsgemäß in drei Abschnitte eingeteilt werden:
Im oberen (dem Niedertemperaturbereich) findet hauptsächlich die Trennung zwischen Edukten und Reaktionswasser statt; im unteren (dem Hochtemperaturbereich) wird das Produkt von unumgesetztem Edukt und Katalysatorresten getrennt, in dem mittleren, dem Reaktionsbereich, werden die Edukte und nach Möglichkeit die Katalysatoren aus den beiden anderen Bereichen zusammengeführt und unter Wasserabspaltung umgesetzt.

Da die Reaktion bestenfalls schwach exotherm ist und das entstehende Wasser mit einem relativ hohen Energieaufwand in die Gasphase gebracht werden muß und dabei entsprechend ihren Partialdampfdrücken Eduktmoleküle mit verdampft werden müssen, benötigt der Reaktionsteil einen Energieeintrag, der im allgemeinen größer ist als die Energie, die zur Abtrennung von unumgesetztem Edukt vom Azomethin benötigt wird.

In einer speziellen Form des Verfahrens wird demnach unterhalb der Reaktionszone ein Wärmetauscher installiert, der die größte Menge unumgesetzten Edukts verdampft, um den isolierten Reaktionsteil mit Energie zu versorgen und den Hochtemperaturbereich hiervon zu entlasten. Gegebenenfalls kann dem Reaktionsteil an geeigneter Stelle zusätzlich nochmals Energie zugeführt werden, im allgemeinen dort, wo der meiste Umsatz vonstatten geht und damit die größte Menge Wasser gebildet wird, Diese zusätzlichen Energieeintragsstellen entlasten nicht nur den Hochtemperaturbereich der Kolonne, sie ermöglichen auch den Eintrag eines beträchtlichen Teils der benötigten Energie auf niedrigem thermischen Niveau.

Oberhalb des Reaktionsteils kann gegebenenfalls ein Wärmetauscher installiert werden, der unumgesetztes Edukt durch Kondensation, wenn möglich unter Energierückgewinnung, vom Reaktionswasser abtrennt und so den Niedertemperaturbereich, in dem die Azeotropbildung stattfindet, entlastet.

Die Auslegung der einzelnen Bereiche ist eine geläufige ingenieurtechnische Aufgabe, die nach bekannter Methodik für den Einzelfall durch Experimente und Rechnungen gelöst wird.

Die drei Abschnitte der Kolonne können entsprechend der Auslegung der Apparatur unterschiedliche Querschnittsflächen besitzen, um den in ihnen herrschenden unterschiedlichen Gas- und Flüssigkeitsbelastungen Rechnung zu tragen.

Ohne Verdampferteil unterhalb der Reaktionszone muß die gesamte für Reaktion und Abtrennung benötigte Energie am Fuß der Kolonne durch Verdampfung von Azomethin in das System gebracht werden und von dort als Verdampfungsenthalpie nach oben transportiert werden, wobei sie nacheinander zur Trennung von Edukt und Produkt und von Wasser und Edukt verwendet wird. Dies bedeutet, daß der untere Volumenteil entsprechend seiner hohen Flüssigkeits- und Gasbelastung wenigstens die gleiche, bevorzugt die 1,5-fache Querschnittsfläche und höchstens die dreifache, bevorzugt maximal die doppelte Querschnittsfläche des Reaktionsteils besitzt.

Wird der Reaktionsteil unten mit einem Verdampfer und oben mit einem Dephlegmator ausgerüstet, sind sowohl Nieder- als auch Hochtemperaturbereich entlastet und besitzen dementsprechend höchstens die gleiche, bevorzugt nur zwei Drittel, jedoch mindestens ein Drittel, bevorzugt mindestens die Hälfte der Querschnittsfläche des Reaktionsteils.

Die Ausmaße hängen nicht zuletzt auch von den gewählten Kolonneneinbauten der unterschiedlichen Kolonnenzonen ab (Füllkörper oder feste Einbauten, welche identisch oder verschieden sein können).

Die zu verwendenden Füllkörper beziehungsweise geordneten Packungen sind die für Destillationen an sich üblichen, wie sie beispielsweise in Ullmann's Encyclopädie der Techn. Chemie Bd. 2, S 528 ff (4. Auflage) oder in den Firmenschriften, z.B. von Sulzer, Montz, Raschig, Kühni oder Norton beschrieben sind.

Genannt seien beispielsweise:
Raschig®- oder Pallringe®, Berl-, Intalox®- oder Torussättel®, Interpackkörper aus verschiedenen Materialien, wie Glas, Steinzeug, Porzellan, Kohlenstoff, Edelstahl, Kunststoff, die (bes. als Metall) gewebe- oder maschenartig verarbeitet sein können.

Bevorzugt sind Füllkörper und geordnete Packungen, die eine große Oberfläche und gute Benetzung sowie eine ausreichende Verweilzeit der Flüssigkeit aufweisen, beispielsweise Pall®- und Novolax®-Ringe, Berlsättel®, BX-Packungen®, Montz-Pak®, Mellapak®, Melladur®, Kerapak®, CY-Packungen®, Ralu-Pak® und Rombo-Pak®.

Für das erfindungsgemäße Verfahren geeignet sind jedoch nicht nur Füllkörperkolonnen, sondern auch insbesondere solche mit festen Einbauten.

Geeignet sind Bodenkolonnen mit Sieb-, Glocken-, Ventil-, Tunnel- und Zentrifugalböden, die wiederum in unterschiedlichen Ausführungen vorliegen können.

Bevorzugt sind z.B. Glocken- und Ventilböden mit hohen Verweilzeiten bei gutem Stoffaustausch; dies gilt insbesondere für den Reaktionsteil der Kolonne.

Das erfindungsgemäße Verfahren wird in einem Druckbereich von 0,5 mbar bis 3 bar, bevorzugt von 1 mbar bis 1 bar, besonders bevorzugt bei 2 - 250 mbar, ganz besonders bevorzugt bei 3 - 180 mbar, durchgeführt. Für die bevorzugte Durchführung im Unterdruckbereich ist der kolonnenartige Reaktor an seinem oberen Niedertemperaturende mit einer Vakuumpumpe ausgerüstet, die vor oder hinter den Kondensator für das azeotrop zu entnehmende Wasser eingeschaltet werden kann.

In der Destillationskolonne baut sich beim Betrieb ein stationäres Temperaturprofil auf. Dieses Temperaturprofil umfaßt den Temperaturbereich von 10 bis 300°C, bevorzugt 15 bis 250°C, besonders bevorzugt 20 bis 200°C.

Der Temperaturbereich der Reaktionszone, die Zone in der mehr als 90 % des Reaktionswassers freigesetzt wird und die gegebenenfalls am oberen Ende mit einem Dephlegmator und am unteren Ende mit einem Verdampfer versehen ist, beträgt bevorzugt 25 bis 180°C, besonders bevorzugt 35 bis 160°C, ganz besonders bevorzugt 45 bis 140°C.

Der angelegte Druck an eine Destillationskolonne bestimmt zusammen mit dem Druckaufbau durch den Strömungswiderstand in der Apparatur die Siedetemperaturen der Flüssigkeitsmischungen im Inneren der Kolonne.

Ist die Umsetzung langsam und wird zur Verbesserung der Raum-Zeit-Ausbeute der Druck im Reaktionsteil und damit die Temperatur im Reaktionsteil angehoben, so steigt gleichzeitig die thermische Belastung für das hochsiedende Reaktionsprodukt am Ende der Kolonne.

Treten hierdurch Probleme, das heißt Zersetzungsreaktionen, auf, so kann gegebenenfalls der Hochtemperaturbereich unterteilt werden, indem man die Temperatur am unteren Ende der Kolonne so wählt, daß noch keine Zersetzung auftritt, jedoch unumgesetztes Anilin und Cycloalkanon mit ausgetragen werden. Diese Mischung wird in einer parallel bei deutlich niedrigerem Druck arbeitenden Kolonne (dem zweiten Teil des Hochtemperaturbereiches) von dem Anilin- und Cycloalkanonresten befreit, welche als Kopfprodukt dieser zweiten Destillationsapparatur entweder nach Kondensation oder direkt nach Komprimierung der Reaktionskolonne zugeführt werden.

Die Einspeisung erfolgt an einer beliebigen Stelle des Reaktionsbereiches (z.B. im ersten Teil des Hochtemperaturbereiches) oder des Niedertemperaturbereiches, bevorzugt an der Stelle, wo das Anilin/Cycloalkanon-Verhältnis der flüssigen Phase in der Reaktionskolonne gleich dem des Kopfproduktes ist.

Der Druck in diesem zweiten Teil des Hochtemperaturbereiches ist bevorzugt 1/1000 bis 1/10, besonders bevorzugt 1/100 bis 1/10 des Arbeitsdruckes der Reaktionskolonne.

Die Eingangsstoffe können einzeln oder als Mischung oberhalb der Reaktionszone an einem beliebigen Punkt im Niedertemperaturbereich in den Reaktor eingespeist werden. Bevorzugt werden sie als Gemisch unmittelbar oberhalb der Reaktionszone eingespeist. In besonders bevorzugter Weise wird das Gemisch an dem Punkt in den Reaktor eingespeist, an dem das Verhältnis der Einsatzstoffe in der flüssigen Phase im Reaktor gleich ist dem Verhältnis der einzuspeisenden Mischung. Für den Fall, daß einer der Einsatzstoffe infolge seiner größeren Flüchtigkeit in der Reaktionszone unterrepräsentiert ist, kann er jedoch ganz oder auch teilweise unterhalb der Reaktionszone oder im unteren Teil der Reaktionszone eingespeist und auf diese Weise dem anderen Reaktionspartner ganz oder teilweise im Gegenstrom entgegengeführt werden.

Wird ein separater Azeotropbildner eingesetzt, kann er ebenfalls getrennt von den Ausgangstoffen eingespeist werden; in bevorzugter Weise wird er jedoch mit der Mischung der Ausgangsstoffe oder zumindest gemischt mit einem der Ausgangsstoffe eingespeist.

Das gleiche gilt für den Katalysator oder die Katalysatormischung, der (die) bevorzugt mit den Edukten gemischt zudosiert wird, jedoch gegebenenfalls auch den Edukten aus dem unteren Teil der Apparatur entgegengeführt werden kann, wobei darauf zu achten ist, daß kein oder nur wenig Katalysator in den untersten Kolonnenbereich gelangt.

Das Azomethin als Reaktionsprodukt wird am unteren Reaktorende im Hochtemperaturbereich, beispielsweise aus dem Sumpfumlauf abgeführt. Das Produkt ist normalerweise sehr rein und kann ohne weitere Reinigung weiter verwendet werden. Für den Fall, daß schwerflüchtige Nebenprodukte abgetrennt werden sollen, kann das gewünschte Azomethin in einer grundsätzlich dem Verfahrenstechniker bekannten Weise an einem Punkt oberhalb der Abnahme solcher schwerflüchtiger Nebenprodukte dem kolonnenartigen Reaktor entnommen werden und zwar dort, wo eine maximale Reinheit gewährleistet ist.

In einer bevorzugten Form wird dem im Hochtemperaturbereich entnommenen Azomethin ein ebenfalls im Hochtemperaturbereich, d.h. in jedem Falle unterhalb der Reaktionszone eingespeister Inertgasstrom entgegengeführt. Als Inertgas sei beispielsweise genannt: Luft, Stickstoff, Argon oder Methan, die alle bevorzugt in getrockneter Form eingesetzt werden.

Am oberen Ende des kolonnenartigen Reaktors, also im Niedertemperaturbereich wird Reaktionswasser als Azeotrop entnommen und in einem Scheidegefäß in eine wäßrige und in eine organische Phase des Azeotropbildners getrennt. Die organische Phase wird in bevorzugter Weise dem Reaktor wieder zugeführt, insbesondere, wenn einer der Reaktionspartner gleichzeitig der Azeotropbildner ist; dies kann an einer der oben genannten Stellen erfolgen.

Mit diesem Kreisstrom wird eine nicht unerhebliche Menge an saurem Katalysator zu der Reaktionzone zurückgeführt. Diese Menge muß bei der Säuredosierung berücksichtigt werden.

Am einfachsten beurteilt man den Säuregehalt einer Apparatur mit Kreisstrom, indem man den pH-Wert der wäßrigen Phase des Destillates mißt und durch den Katalysatoreintrag konstant hält.

Der Vorteil des erfindungsgemäßen Verfahrens gegenüber dem Stand der Technik besteht in der Möglichkeit des nahezu äquimolaren Einsatzes der Ausgangsstoffe, die sodann vollständig in einem einzigen Durchgang umgewandelt werden können. Hierbei wird unter Vermeidung von zusätzlichen Aufarbeitungsschritten eine quantitative Ausbeute erzielt. Das anzuwendende Verfahren ist durch große Einfachheit und durch optimale Ausnutzung der eingesetzten Energie ausgezeichnet.

Die erfindungsgemäß herstellbaren Azomethine sind Ausgangsstoffe für die Hydrierung zu Cyclohexyl-cycloalkylaminen und Phenyl-cycloalkylaminen und für den Fall, daß das Cycloalkanon ein Cyclohexanon ist, zur Dehydrierung zu gegebenenfalls substituierten Diphenylaminen.

### Beispiel 1

Die Apparatur besteht von unten nach oben aus: Glaskolben (250 ml) mit geregelter Stickstoffeinleitung zum Aufnehmen des Sumpfproduktes (aus diesem Kolben wird das Azomethin gewonnen, indem es von Zeit zu Zeit abgesaugt wird); ölthermostatisierter Schlangenverdampfer (Schlangenrohrlänge ca. 1,50 m, Rohrinnendurchmesser ca. 1,5 cm); vakuumverspiegelte Füllkörperkolonne, gefüllt mit 0,5 cm großen Porzellansattelkörpern (Innendurchmesser der Kolonne ca. 2,7 cm, Höhe der Kolonne ca. 35 cm); ölthermostatisierter Kolonnenteil, gefüllt mit Porzellansattelkörpern (Durchmesser ca. 2,7 cm, Höhe ca. 35 cm); ölthermostatisierte Siebbodenkolonne (Innendurchmesser ca. 5 cm, Höhe ca. 60 cm, 10 Böden mit zusammen ca. 60 ml Flüssigkeitfüllung, der Ölmantel wurde auf eine Temperatur thermostatisiert, die der Innentemperatur der Kolonne an allen Stellen möglichst nahe kam); Eindosierschuß (Innendurchmesser ca. 2,7 cm, Höhe ca. 5 cm); Ölthermostatisierter Kolonnenteil mit Porzellansattelkörpern (Durchmesser (innen) ca. 2,7 cm, Höhe ca. 15 cm); vakuumverspiegelte Füllkörperkolonne mit Porzellansattelkörpern (Innendurchmesser ca. 2,7 cm, Höhe ca. 25 cm); Luftbrücke zum abwärtsbetriebenen Kondensationsteil, bestehend aus zwei Intensivkühlern, Anschützaufsatz, Vakuumentnahmestutzen und Gaskappe mit Vakuumschlauch.

Das Vakuum in der Apparatur wurde durch eine Membranpumpe aufrecht erhalten; gesteuert wurde die Evakuierung durch einen Vakuumkontroller mit regelbarem Kugelschottventil (± 0,5 mbar).

Die Eduktmischdosierung erfolgt durch eine vakuumdichte Dosierpumpe (TELAP, PTFE-Minidosierer). Die vier thermostatisierten Kolonnenbereiche wurden mittels Ölthermostaten beheizt, gekühlt beziehungsweise isoliert.

Der Druck wurde auf 60 mbar eingestellt und ein N₂-Strom von 0,1 Nl/h in den unteren Teil der Apparatur gegeben. Nachdem die Ölthermostate ihre Solltemperatur erreicht hatten (Öltemperatur unterer Verdampfer ca. 195°C, Öltemperatur mittlerer Verdampfer ca. 190°C, Öltemperatur Siebbodenkolonne ca. 80°C, Öltemperatur oberer Kondensator ca. 20°C) wurde die Eduktdosierung in Gang gesetzt (330 ml/h). Das Edukt bestand aus 1,2 Molen Cyclohexanon, 1 Mol Anilin und 1 Mol-% (bezogen auf beiden Komponenten) Essigsäure (117,6 g, 93 g, 1,32 g).

Nach ca. 1 bis 2 h Laufzeit war die Apparatur im Gleichgewicht und am Fuß der Kolonne verließen pro Stunde ca. 264 g Cyclohexylidenanilin mit einer Reinheit von besser als 99,9 % den Schlangenverdampfer. Der Essigsäuregehalt des Azomethins (Anils) lag unterhalb von 10 ppm (Bestimmung mittels Ionenchromatographie).

Am Kopf der Kolonnen kondensierten ca. 59 g einer Cyclohexanon-Wassermischung, die mit einem Volumenverhältnis von 1,1 zu 1 in eine organische und eine wäßrige Phase aufspaltete` Diese Mischung enthielt den Katalysator.

### Beispiel 2

Wie Beispiel 1 wurde verfahren, jedoch wurden 0,2 mol-% (264 mg) Essigsäure und 0,0004 mol-% (0,65 mg) Propionsäure der Eduktmischung aus 1,2 Molen Cyclohexanon und 1 Mol Anilin zugemischt. Der Versuch verlief, wie in Beispiel 1 beschrieben, jedoch wurde das Gleichgewicht erst nach etwas längerer Laufzeit vollständig eingestellt. Das Cyclohexylidenanilin (Reinheit besser als 99,9 %) enthielt ebenfalls weniger als 10 ppm Essigsäure und weniger als 0,5 ppm (untere Nachweisgrenze) an Propionsäure.

### Beispiel 3

Senkte man den Katalysatorgehalt von Beispiel 2 weiter, so erfolgte die Einstellung des chemischen Gleichgewichts für die beschriebene Belastung zu langsam, so daß der größte Teil der zudosierten Eduktmischung über Kopf abdestillierte.

### Beispiel 4

Wie Beispiel 2, jedoch ohne Propionsäure. Verlauf analog Beispiel 3.

### Beispiel 5

Wie Beispiel 1, jedoch wurde 1 Mol-% wasserfreie HCl (803 mg) der Eduktmischung aus 1,2 Molen Cyclohexanon und 1 Mol Anilin zugemischt.

Der Verlauf des Beispiels entsprach weitgehend dem Verlauf des Beispiels 1, allerdings mit dem Unterschied, daß die Temperatur im unteren Kolonnenteil um 20 bis 30°C unterhalb der im Beispiel 1 lag und als Produkt eine Mischung aus 5 % nicht identifizierten Hochsiedern, 48 % Cyclohexenyl-cyclohexylidenanilin, 25 % Cyclohexylidenanilin, 19 % Anilin und 3 % Cyclohexanon anfiel.

## Patentansprüche

1. Verfahren zur Herstellung von Azomethinen der Formel 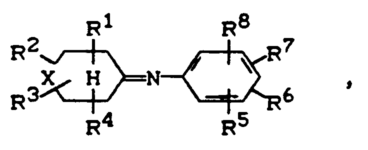 in der
R¹, R², R³, R⁴, R⁵, R⁶, R⁷ und R⁸ unabhängig voneinander Wasserstoff, geradkettiges oder verzweigtes C₁-C₆-Alkyl, C₃-C₆-Cycloalkyl, Benzyl oder Aryl bedeuten und
R⁵, R⁶, R⁷ und R⁸ unabhängig voneinander zusätzlich Halogen, geradkettiges oder verzweigtes C₁-C₆-Alkoxy, Hydroxy, Amino, C₁-C₆-Alkylamino, Di-C₁-C₆-alkyl-amino, Aryloxy oder Arylamino bedeuten,
wobei Aryl für Phenyl oder für in 2-, 3- oder 4-Stellung gebundenes 5- oder 6-gliedriges Heteroaryl mit 1 oder 2 Heteroatomen aus der Gruppe N, O und S steht, und
X -CH₂- oder eine zwischen den benachbarten C-Atomen durchgehende Bindung bedeutet,
durch Kondensation von Cycloalkanonen der Formel 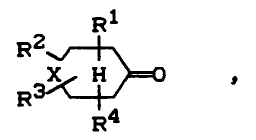 mit Anilinen der Formel 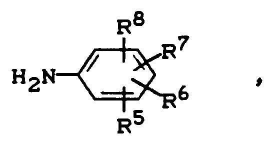 in denen R¹ bis R⁸ und X die obige Bedeutung haben,
dadurch gekennzeichnet, daß die Kondensation in kontinuierlicher Reaktion in einer Destillationskolonne in Gegenwart von sauren homogenen Katalysatoren mit einem in H₂O gemessenen pkₐ-Wert von 1-6 unter azeotroper Entfernung des Reaktionswassers, durchgeführt wird, wobei die Ausgangsstoffe, der Azeotropbildner und der Katalysator oder die Katalysatormischung getrennt an verschiedenen Orten oder zusammen als Mischung am gleichen Ort in die Destillationskolonne eingespeist werden und von den Reaktionsprodukten das azeotrop zu entfernende Reaktionswasser aus dem Niedertemperaturbereich und das entstehende Azomethin aus dem Hochtemperaturbereich abgeführt werden.

2. Verfahren nach Anspruch 1, dadurch gekennzeichnet, daß Cycloalkanone der Formel 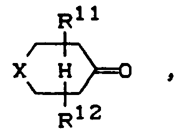 eingesetzt werden, worin
R¹¹ und R¹² unabhängig voneinander Wasserstoff, geradkettiges oder verzweigtes C₁-C₄-Alkyl, Cyclopropyl, Cyclopentyl, Cyclohexyl, Benzyl oder Phenyl bedeuten und
X -CH₂- oder eine zwischen den benachbarten C-Atomen durchgehende Bindung bedeutet
und bevorzugt Cycloalkanone der Formel 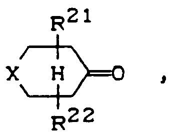 eingesetzt werden, worin
R²¹ und R²² unabhängig voneinander Wasserstoff, Methyl oder Ethyl bedeuten und
X die obige Bedeutung hat.

3. Verfahren nach Anspruch 1, dadurch gekennzeichnet, daß die Cycloalkanone Cyclohexanone sind.

4. Verfahren nach Anspruch 1, dadurch gekennzeichnet, daß Aniline der Formel 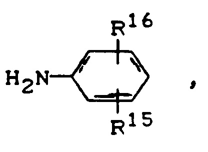 eingesetzt werden, in der
R¹⁵ und R¹⁶ unabhängig voneinander Wasserstoff, geradkettiges oder verzweigtes C₁-C₄-Alkyl, Phenyl, Fluor, Chlor, Brom, geradkettiges oder verzweigtes C₁-C₄-Alkoxy, Hydroxy, Amino, C₁-C₄-Alkylamino, Di-C₁-C₄-alkyl-amino, Phenoxy oder Phenylamino bedeuten
und bevorzugt Aniline der Formel 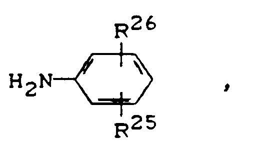 eingesetzt werden, in der
R²⁵ und R²⁶ unabhängig voneinander Wasserstoff, Methyl, Ethyl, Fluor, Chlor, Methoxy, Ethoxy, Methylamino, Ethylamino, Dimethylamino oder Diethylamino bedeuten
und besonders bevorzugt Aniline der Formel 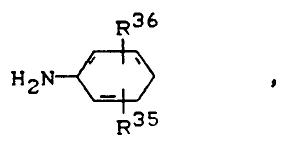 eingesetzt werden, in der
R³⁵ und R³⁶ unabhängig voneinander Wasserstoff, Fluor, Chlor, Methyl, Ethyl, Methoxy, Methylamino oder Dimethylamino bedeuten.

5. Verfahren nach Anspruch 1, dadurch gekennzeichnet, daß das Cycloalkanon und das Anilin im molaren Verhältnis von 2:1-1:2, bevorzugt 1,5:1-1:1,5, besonders bevorzugt 1,2:1-1:1,2, ganz besonders bevorzugt 1,1:1-1:1,1 eingesetzt werden.

6. Verfahren nach Anspruch 1, dadurch gekennzeichnet, daß in den kolonnenartigen Reaktor im Verhältnis zur Summe der dosierten Eduktmoleküle saurer Katalysator beziehungsweise saure Katalysatormischung im Bereich von 10 bis 0,001 mol-%, bevorzugt von 1 bis 0,01 mol-%, besonders bevorzugt von 0,5 bis 0,02 mol-% dosiert wird.

7. Verfahren nach Anspruch 1, dadurch gekennzeichnet, daß das Temperaturprofil im kolonnenartigen Reaktor im Bereich von 10 bis 300°C, bevorzugt von 15 bis 250°C, besonders bevorzugt von 20 bis 200°C liegt.

8. Verfahren nach Anspruch 1, dadurch gekennzeichnet, daß dem im Hochtemperaturbereich entnommenen Azomethin ein ebenfalls im Hochtemperaturbereich eingespeister Inertgastrom entgegengeführt wird.

9. Verfahren nach Anspruch 1, dadurch gekennzeichnet, daß bei einem Druck von 0,5 mbar bis 3 bar, bevorzugt 1 mbar bis 1 bar, besonders bevorzugt 3 bis 180 mbar gearbeitet wird.

10. Verfahren nach Anspruch 1, dadurch gekennzeichnet, daß als saure homogene Katalysatoren anorganische oder organische Verbindungen eingesetzt werden, die einen in Wasser gemessenen pkₐ-Wert von 1 bis 6, bevorzugt von 2 bis 5,5, besonders bevorzugt von 3 bis 5 aufweisen, und die bevorzugt Alkyl- beziehungsweise Arylcarbonsäuren mit 1 bis 10, bevorzugt 2 bis 6 C-Atomen sind.

## Claims

1. Method for the preparation of azomethines of the formula 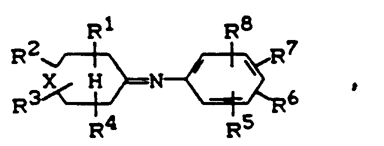 in which
R¹, R², R³, R⁴, R⁵, R⁶, R⁷ and R⁸, independently of one another, represent hydrogen, straight-chain or branched C₁-C₆-alkyl, C₃-C₆-cycloalkyl, benzyl or aryl and
R⁵, R⁶, R⁷ and R⁸, independently of one another, additionally represent halogen, straight-chain or branched C₁-C₆-alkoxy, hydroxyl, amino, C₁-C₆-alkylamino, di-C₁-C₆-alkyl-amino, aryloxy or arylamino,
in which aryl represents phenyl or 5- or 6-membered heteroaryl having 1 or 2 heteroatoms from the group N, O and S which is linked in the 2-, 3- or 4-position, and
X represents -CH₂- or a bond connecting the neighbouring C atoms,
by condensation of cycloalkanones of the formula 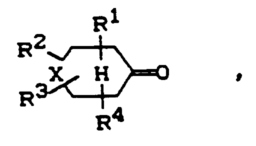 with anilines of the formula 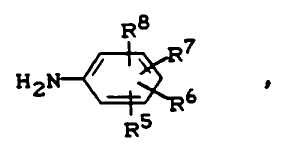 in which R¹ to R⁸ and X have the abovementioned meaning,
characterised in that the condensation is carried out in a continuous reaction in a distillation column in the presence of acid homogeneous catalysts having a pKₐ value measured in H₂O of 1 to 6, while the water of reaction is removed azeotropically, the starting materials, the azeotropic agent and the catalyst or the catalyst mixture being fed separately at different locations, or together as a mixture at the same location, into the distillation column, and, of the reaction products, the water of reaction to be removed azeotropically being discharged from the low-temperature region and the azomethine produced being discharged from the high-temperature region.

2. Method according to Claim 1, characterised in that cycloalkanones of the formula 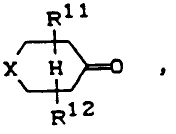 are used in which
R¹¹ and R¹², independently of one another, represent hydrogen, straight-chain or branched C₁-C₄-alkyl, cyclopropyl, cyclopentyl, cyclohexyl, benzyl or phenyl and
X represents -CH₂- or a bond connecting the neighbouring C atoms,
and preferably cycloalkanones of the formula 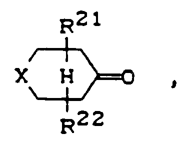 are used in which
R²¹ and R²², independently of one another, represent hydrogen, methyl or ethyl and
X has the abovementioned meaning.

3. Method according to Claim 1, characterised in that the cycloalkanones are cyclohexanones.

4. Method according to Claim 1, characterised in that anilines of the formula 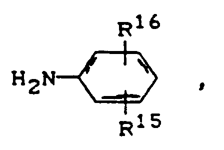 are used in which
R¹⁵ and R¹⁶, independently of one another, represent hydrogen, straight-chain or branched C₁-C₄-alkyl, phenyl, fluorine, chlorine, bromine, straight-chain or branched C₁-C₄-alkoxy, hydroxyl, amino, C₁-C₄-alkylamino, di-C₁-C₄-alkyl-amino, phenoxy or phenylamino
and preferably anilines of the formula 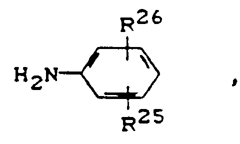 are used in which
R²⁵ and R²⁶, independently of one another, represent hydrogen, methyl, ethyl, fluorine, chlorine, methoxy, ethoxy, methylamino, ethylamino, dimethylamino or diethylamino
and, particularly preferably, anilines of the formula 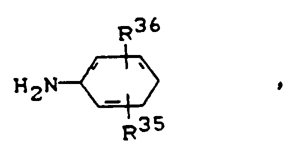 are used in which
R³⁵ and R³⁶, independently of one another, represent hydrogen, fluorine, chlorine, methyl, ethyl, methoxy, methylamino or dimethylamino.

5. Method according to Claim 1, characterised in that the cycloalkanone and the aniline are used in a molar ratio of 2:1-1:2, preferably 1.5:1-1:1.5, particularly preferably 1.2:1-1:1.2, most particularly preferably 1.1:1-1:1.1.

6. Method according to Claim 1, characterised in that acid catalyst or acid catalyst mixture is metered into the column-type reactor in a ratio, based on the sum of the metered starting material molecules, in the range from 10 to 0.001 mol%, preferably from 1 to 0.01 mol%, particularly preferably from 0.5 to 0.02 mol%.

7. Method according to Claim 1, characterised in that the temperature profile in the column-type reactor is in the range from 10 to 300°C, preferably from 15 to 250°C, particularly preferably from 20 to 200°C.

8. Method according to Claim 1, characterised in that, in counterflow to the azomethine withdrawn in the high-temperature region, an inert gas flow is fed in, also in the high-temperature region.

9. Method according to Claim 1, characterised in that a pressure from 0.5 mbar to 3 bar, preferably 1 mbar to 1 bar, particularly preferably 3 to 180 mbar is used.

10. Method according to Claim 1, characterised in that, as acid homogenous catalysts, inorganic or organic compounds are used which have a pKₐ value, measured in water, from 1 to 6, preferably from 2 to 5.5, particularly preferably from 3 to 5, and which are preferably alkylcarboxylic or arylcarboxylic acids having 1 to 10, preferably 2 to 6 C atoms.

## Revendications

1. Procédé pour la préparation d'azométhines répondant à la formule 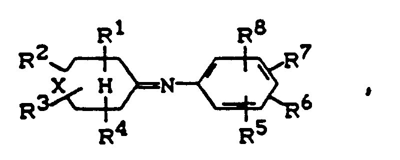 dans laquelle
R¹, R², R³, R⁴, R⁵, R⁶, R⁷ et R⁸ représentent, indépendamment l'un de l'autre, un atome d'hydrogène, un groupe alkyle en C₁-C₆ à chaîne droite ou ramifiée, un groupe cycloalkyle en C₃-C₆, un groupe benzyle ou un groupe aryle, et
R⁵, R⁶, R⁷ et R⁸ représentent, indépendamment l'un de l'autre, en outre, un atome d'halogène, un groupe alcoxy en C₁-C₆ à chaîne droite ou ramifiée, un groupe hydroxyle, un groupe amino, un groupe alkyl(en C₁-C₆)amino, un groupe di(alkyl en C₁-C₆)amino, un groupe aryloxy ou un groupe arylamino,
le terme "aryle" désignant un groupe phényle ou un groupe hétéroaryle penta- ou hexagonal lié en position 2, 3 ou 4, contenant 1 ou 2 hétéroatomes choisis parmi le groupe comprenant N, O et S, et
X représente -CH₂- ou encore une liaison continue entre les atomes de carbone voisins,
par condensation de cycloalcanones répondant à la formule 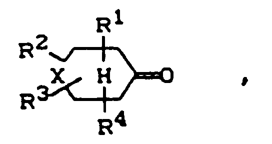 avec des anilines répondant à la formule 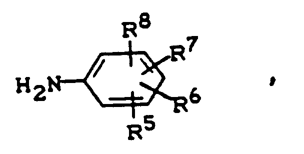 dans lesquelles R¹ à R⁸ et X ont la signification indiquée ci-dessus,
caractérisé en ce qu'on effectue la condensation en une réaction en continu dans une colonne de distillation en présence de catalyseurs homogènes acides dont la valeur pKₐ mesurée dans H₂O est de 1-6, avec élimination azéotrope de l'eau réactionnelle, les substances de départ, le formateur d'azéotrope et le catalyseur ou le mélange de catalyseurs étant alimentés de manière séparée à des endroits différents ou de manière conjointe en mélange au même endroit dans la colonne de distillation, et l'eau réactionnelle à séparer par voie azéotrope des produits réactionnels étant évacuée de la zone à basse température, et l'azométhine qui se forme étant évacuée de la zone à température élevée.

2. Procédé selon la revendication 1, caractérisé en ce qu'on met en oeuvre des cycloalcanones répondant à la formule 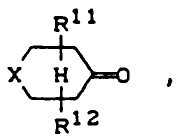 dans laquelle
R¹¹ et R¹² représentent, indépendamment l'un de l'autre, un atome d'hydrogène, un groupe alkyle en C₁-C₄ à chaîne droite ou ramifiée, un groupe cyclopropyle, un groupe cyclopentyle, un groupe cyclohexyle, un groupe benzyle ou un groupe phényle, et
X représente -CH₂- ou une liaison continue entre les atomes de carbone voisins,
et on met en oeuvre de préférence des cycloalcanones répondant à la formule 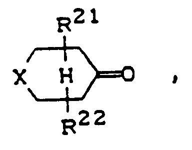 dans laquelle
R²¹ et R²² représentent, indépendamment l'un de l'autre, un atome d'hydrogène, un groupe méthyle ou un groupe éthyle, et
X a la signification indiquée ci-dessus.

3. Procédé selon la revendication 1, caractérisé en ce que les cycloalcanones sont des cyclohexanones.

4. Procédé selon la revendication 1, caractérisé en ce qu'on met en oeuvre des anilines répondant à la formule 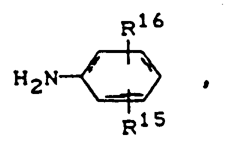 dans laquelle
R¹⁵ et R¹⁶ représentent, indépendamment l'un de l'autre, un atome d'hydrogène, un groupe alkyle en C₁-C₄ à chaîne droite ou ramifiée, un groupe phényle, un atome de fluor, un atome de chlore, un atome de brome, un groupe alcoxy en C₁-C₄ à chaîne droite ou ramifiée, un groupe hydroxyle, un groupe amino, un groupe alkyl(en C₁-C₄)amino, un groupe di(alkyl en C₁-C₄)amino, un groupe phénoxy ou un groupe phénylamino
et on met en oeuvre de préférence des anilines répondant à la formule 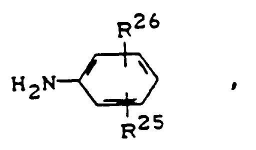 dans laquelle
R²⁵ et R²⁶ représentent, indépendamment l'un de l'autre, un atome d'hydrogène, un groupe méthyle, un groupe éthyle, un atome de fluor, un atome de chlore, un groupe méthoxy, un groupe éthoxy, un groupe méthylamino, un groupe éthylamino, un groupe diméthylamino ou un groupe diéthylamino,
et on met en oeuvre de manière particulièrement préférée des anilines répondant à la formule 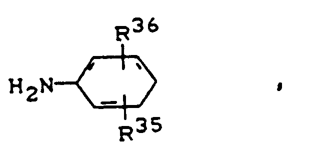 dans laquelle
R³⁵ et R³⁶ représentent, indépendamment l'un de l'autre, un atome d'hydrogène, un atome de fluor, un atome de chlore, un groupe méthyle, un groupe éthyle, un groupe méthoxy, un groupe méthylamino ou un groupe diméthylamino.

5. Procédé selon la revendication 1, caractérisé en ce qu'on met en oeuvre la cycloalcanone et l'aniline dans un rapport molaire de 2:1-1:2, de préférence de 1,5:1-1:1,5, de manière particulièrement préférée de 1,2:1-1:1,2, de manière tout particulièrement préférée de 1,1:1-1:1,1.

6. Procédé selon la revendication 1, caractérisé en ce qu'on dose, dans le réacteur du type à colonne, par rapport à la somme des molécules déduit dosées, un catalyseur acide, respectivement un mélange de catalyseurs acides dans le domaine de 10 à 0,001 mole %, de préférence de 1 à 0,01 mole %, de manière particulièrement préférée de 0,5 à 0,02 mole %.

7. Procédé selon la revendication 1, caractérisé en ce que le profil de température dans le réacteur de type à colonne se situe dans le domaine de 10 à 300°C, de préférence dans le domaine de 15 à 250°C, de manière particulièrement préférée dans le domaine de 20 à 200°C.

8. Procédé selon la revendication 1, caractérisé en ce qu'on guide, à contre-courant de l'azométhine récupérée de la zone à température élevée, un courant de gaz inerte également alimenté dans la zone à température élevée.

9. Procédé selon la revendication 1, caractérisé en ce qu'on travaille sous une pression de 0,5 mbar à 3 bar, de préférence de 1 mbar à 1 bar, de manière particulièrement préférée de 3 à 180 mbar.

10. Procédé selon la revendication 1, caractérisé en ce que, comme catalyseurs acides homogènes, on met en oeuvre des composés inorganiques ou organiques qui présentent une valeur pKₐ mesurée dans de l'eau de 1 à 6, de préférence de 2 à 5,5, de manière particulièrement préférée de 3 à 5, et qui représentent de préférence des acides alkyl-, respectivement arylcarboxyliques contenant de 1 à 10, de préférence de 2 à 6 atomes de carbone.
